# EUROPEAN PATENT APPLICATION

(11) **EP 4 505 939 A1**
(43) Date of publication of application: **12.02.2025**
(21) Application number: 22936599.4
(22) Date of filing: 26.12.2022
(51) Int. Cl.: A61B 3/10

(54) **ESTIMATION SYSTEM, ESTIMATION METHOD, AND PROGRAM**

(30) Priority: 08.04.2022 JP 2022064626
(71) Applicant: Tomey Corporation, Nagoya-shi, Aichi-ken 451-0051 (JP)
(72) Inventor: HIGASHITA, Risa, Nagoya-shi, Aichi 451-0051 (JP); OKAMOTO, Keiichiro, Nagoya-shi, Aichi 451-0051 (JP)
(74) Representative: BSB Patentanwälte Schütte & Engelen Partnerschaft mbB
(86) International application number: PCT/JP2022/047886
(87) International publication number: WO 2023/195204

(57) **Abstract**

An object is to estimate the age of an eye to be examined.

Provided are: an acquisition module configured to acquire a measurement value of an attribute of an eye to be examined, the attribute being at least one of a plurality of characteristics of an anterior segment and a plurality of dimension values of the eye to be examined; and a determination module configured to determine an estimated age of the eye to be examined on the basis of the measurement value and correspondence information indicating a correspondence between a value of the attribute and an age of an eyeball.

## Description

### TECHNICAL FIELD

The present invention relates to an estimation system, an estimation method, and a program.

### BACKGROUND ART

There is a conventional technique for supporting examination of an eye to be examined. Patent Literature 1 discloses a technique for evaluating the narrowness of a corner angle on the basis of a tomographic image of an anterior segment. It is known that many ocular measurement values including a corner angle have characteristics that change with aging. For example, the corner angle tends to become narrower with aging.

### CITATIONS LIST

### PATENT LITERATURE

Patent Literature 1: JP 2015-43814 A

### SUMMARY OF INVENTION

### TECHNICAL PROBLEMS

A need exists for estimation of the age of an eye to examined from the characteristics of the eye to be examined. For example, the narrowness of the corner angle is evaluated by the value of an angle opening distance (AOD) or the like. Because many of values representing the state of an eye such as an AOD are not familiar to patients, it is sometimes difficult to convey the state of an eye to be examined to a patient when a doctor explains the state of the eye to be examined using such a value. In such a case, it is considered that being able to estimate the age of the eye to be examined enables explanation of the state of the eye using the estimated age so that the patient can easily understand the state of the eye. However, the conventional techniques cannot estimate the age of the eye to be examined.

The present invention has been made in view of the above problem, and an object of the present invention is to estimate the age of an eye to be examined.

### SOLUTIONS TO PROBLEMS

In order to achieve the above object, an estimation system of the present invention includes: an acquisition module configured to acquire a measurement value of an attribute of an eye to be examined, the attribute being at least one of a plurality of characteristics of an anterior segment and a plurality of dimension values of the eye to be examined; and a determination module configured to determine an estimated age of the eye to be examined on the basis of the measurement value and correspondence information indicating a correspondence between a value of the attribute and an age of an eyeball.

As described above, according to the present invention, the estimated age of the eye to be examined corresponding to the measurement value of the attribute of the eye to be examined is obtained from the correspondence information. As a result, the estimation system can determine the estimated age of the eye to be examined.

### BRIEF DESCRIPTION OF DRAWINGS

Fig. 1 is a configuration diagram of an estimation system.
Fig. 2 is a diagram illustrating a scanning-alignment optical system.
Fig. 3 is a configuration diagram of a control unit.
Fig. 4 is an explanatory view of radial scanning.
Fig. 5 is an explanatory view of a TISA.
Fig. 6 is a flowchart illustrating estimation processing.
Fig. 7 is a drawing illustrating an image around a lens.

### DESCRIPTION OF EMBODIMENTS

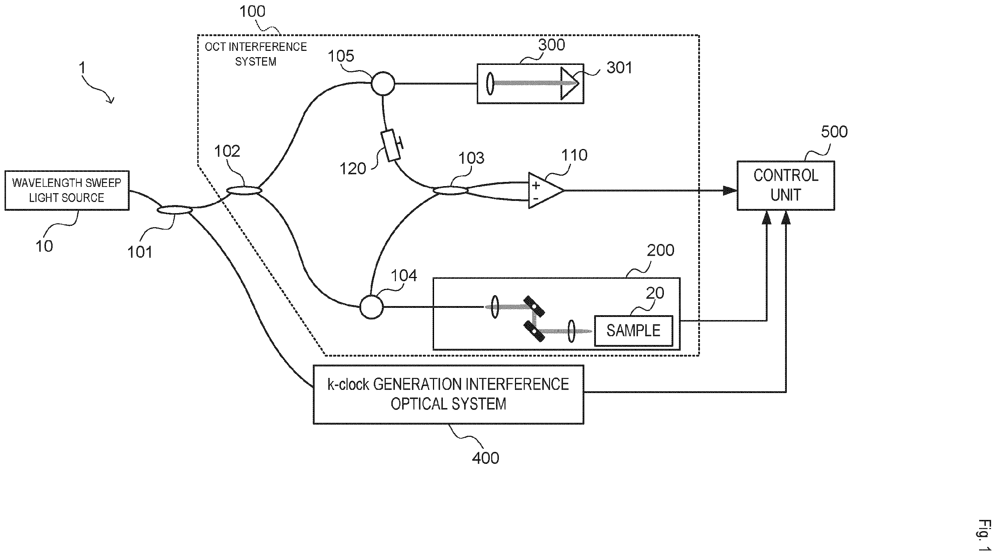

Hereinafter, an estimation system 1 according to the present embodiment will be described. In the present embodiment, the estimation system 1 is an ophthalmic apparatus, and is an optical coherence tomography apparatus that obtains a tomographic image of the anterior segment of an eye to be examined by optical coherence tomography. Fig. 1 is an overall configuration diagram of the estimation system 1. The estimation system 1 mainly includes a wavelength sweep light source 10, a single mode fiber coupler (SMFC) 101, an OCT interference system 100, a k-clock generation interference optical system 400, and a control unit 500. Here, the control unit 500 is an example of a tomographic image processing unit.

The OCT interference system 100 is an optical system for obtaining a tomographic image of the anterior segment of the eye to be examined by the optical coherence tomography. In the present embodiment, an example in which swept source-OCT (SS-OCT) is adopted as the optical coherence tomography will be described. The wavelength sweep light source 10 is a light source that outputs light while performing scanning by changing the wavelength with time. As the wavelength sweep light source 10, for example, a light source having performance capable of achieving high-speed scanning at 50 KHz or more, having a band with a center wavelength of 1 µm or more and a sweep width of 70 nm or more, is used. The light emitted from the wavelength sweep light source 10 is guided by an optical fiber such as a single mode fiber to be used for tomographic imaging of a sample 20 and also used for generation of a k-clock. In the present embodiment, the sample 20 is the eyeball (eye to be examined) of a subject.

The light emitted from the wavelength sweep light source 10 is branched by the SMFC 101 to enter the OCT interference system 100 and the k-clock generation interference optical system 400.

The OCT interference system 100 includes SMFCs 102 and 103, a measurement-side circulator 104, a reference-side circulator 105, a balanced detector 110, a polarization controller 120, a scanning-alignment optical system 200, and a reference optical system 300. One ray of the incident light branched by the SMFC 101 is further branched by the SMFC 102. The other light ray enters the scanning-alignment optical system 200, and the other light ray enters the reference optical system 300.

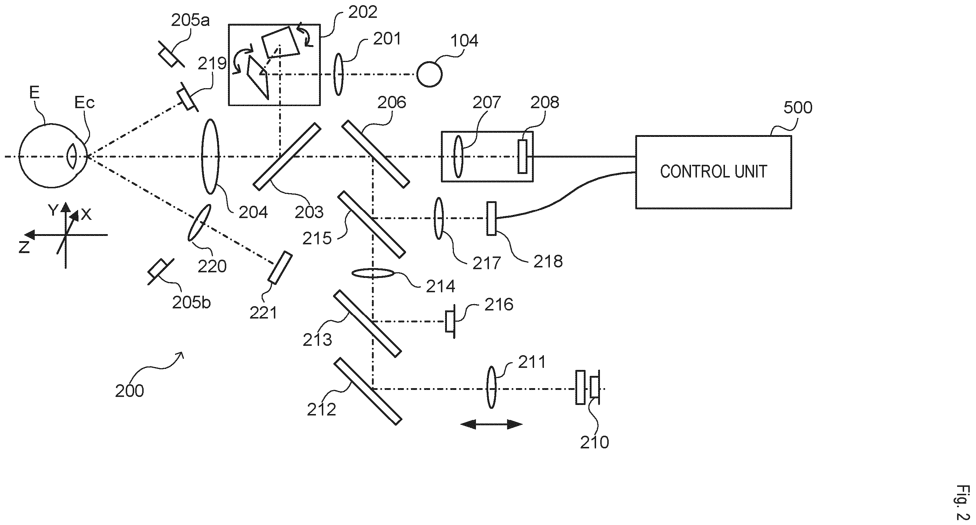

The one light ray branched by the SMFC 102 enters the scanning-alignment optical system 200 through the measurement-side circulator 104. The measurement-side circulator 104 is an optical element disposed among the SMFC 102, the scanning-alignment optical system 200, and the SMFC 103. The scanning-alignment optical system 200 is an optical system that irradiates the sample 20 with incident light and guides reflected light from the sample 20 to the SMFC 103 through the measurement-side circulator 104. The scanning-alignment optical system 200 will be described in detail later with reference to Fig. 2.

The other light ray branched by the SMFC 102 enters the reference optical system 300 through the reference-side circulator 105. The reference-side circulator 105 is an optical element disposed among the SMFC 102, a reference section 301, and the SMFC 103. The reference optical system 300 is provided with the reference section 301 that converts incident light into reference light. In the present embodiment, the reference section 301 is a prism that emits the incident light as the reference light. The reference section 301 is movably provided so as to match an optical path length of the reference optical system 300 with an optical path length of the scanning-alignment optical system 200 before measurement of the sample 20. Note that the position of the reference section 301 is fixed during the measurement of the sample 20.

The light incident on the reference optical system 300 becomes the reference light by the reference section 301, and is guided to the SMFC 103 through the reference-side circulator 105 and the polarization controller 120. The polarization controller 120 is an element that controls polarization of the reference light guided from the reference optical system 300 to the SMFC 103. As the polarization controller 120, controllers of various modes such as an in-line type and a paddle type can be used.

The SMFC 103 combines the reflected light guided from the scanning-alignment optical system 200 and the reference light guided from the reference optical system 300 to generate measurement interference light. In addition, the SMFC 103 branches the combined measurement interference light into two rays of measurement interference light out of phase by 180° and guides the two rays of measurement interference light to the balanced detector 110.

The balanced detector 110 receives the measurement interference light and outputs a measurement interference signal. The measurement interference signal is input to the control unit 500. The control unit 500 generates a tomographic image of the sample 20 from the measurement interference signal by performing arithmetic processing.

Fig. 2 illustrates the configuration of the scanning-alignment optical system 200. The scanning-alignment optical system 200 includes a scanning optical system, an anterior segment photographing system, a fixation target optical system, and an alignment optical system.

The scanning optical system is an optical system for obtaining a tomographic image. In the scanning optical system, light passing through the optical fiber from the measurement-side circulator 104 enters a galvano scanner 202 through a collimator lens 201. The galvano scanner 202 is a device for moving incident light, and is driven by a galvano driver (not illustrated). The light passing through the galvano scanner 202 is reflected by a hot mirror 203 at an angle of 90°, passes through an objective lens 204, and enters an eye to be examined E that is the sample 20. The light incident on the eye to be examined E is reflected at each tissue portion (cornea, anterior chamber, iris, lens, etc.) of an anterior segment Ec to become measurement light. Then, the measurement light passes through the objective lens 204, the hot mirror 203, the galvano scanner 202, and the collimator lens 201 in order reversed from the above, and is guided to the SMFC 103 through the measurement-side circulator 104.

The SMFC 103 combines the reflected light (measurement light) from the anterior segment Ec and the reference light, and a signal thereof is input to the balanced detector 110. The balanced detector 110 measures interference for each wavelength, and the measured measurement interference signal is input to the control unit 500. The control unit 500 performs processing such as inverse Fourier transform on the measurement interference signal to acquire a tomographic image of the anterior segment Ec along a scanning line.

The anterior segment photographing system is a photographing system for obtaining a two-dimensional image of the anterior segment. The anterior segment photographing system includes illumination light sources 205a and 205b, the objective lens 204, the hot mirror 203, a beam splitter 206, an image forming lens 207, and an area sensor 208. The illumination light sources 205a and 205b irradiate the front surface of the eye to be examined E with illumination light in a visible light region. The illumination light is reflected at the eye to be examined E, and the reflected light is guided to the area sensor 208 through the objective lens 204, the hot mirror 203, the beam splitter 206, and the image forming lens 207. As a result, a front image of the anterior segment of the eye to be examined E is photographed, and the photographed front image is input to the control unit 500. Here, the front image is a two-dimensional image of the anterior segment as viewed from the optical axis direction of the reflected light, and is an image whose left-right direction coincides with the left and right of the subject, and whose vertical direction coincides with the up-down direction of the subject.

The fixation target optical system is an optical system for preventing the subject from moving the eyeball (eye to be examined E) as much as possible by looking at a fixation lamp. The fixation target optical system includes a fixation target light source 210, a variable focus movable lens 211, a cold mirror 212, a hot mirror 213, a relay lens 214, a beam splitter 215, the beam splitter 206, the hot mirror 203, and the objective lens 204. Emitted light from the fixation target light source 210 reaches the eye to be examined E through the variable focus movable lens 211, the cold mirror 212, the hot mirror 213, the relay lens 214, the beam splitter 215, the beam splitter 206, the hot mirror 203, and the objective lens 204 in this order.

Here, the variable focus movable lens 211 is configured to be movable so that the focus of the fixation target can be freely changed. For example, the variable focus movable lens 211 can be moved so that the fixation target comes into focus at the position of a refractive power value of the eye to be examined E, thereby achieving a subject's natural viewing state (a state in which no load is applied to the lens).

The alignment optical system is an optical system for positioning of the eye to be examined E. Although not illustrated, the estimation system 1 includes an apparatus main body and a holding table that supports the apparatus main body. A chin receiving portion on which the subject places the chin and a forehead contact portion with which the subject brings the forehead into contact are fixedly provided on the front side (subject side) of the apparatus main body. When the forehead is brought into contact with the forehead contact portion, the eye (eye to be examined E) of the subject is disposed in front of an examination window provided on the front surface of the apparatus main body.

Furthermore, the apparatus main body is supported so as to be movable in an X direction, a Y direction, and a Z direction with respect to the holding table. The apparatus main body can move in each of the X direction, the Y direction, and the Z direction with respect to the holding table under the control of the control unit 500. Here, the X direction corresponds to the left-right direction of the apparatus main body and corresponds to the left-right direction of the head of the subject fixed to the apparatus. The Y direction corresponds to the up-down direction of the apparatus main body and corresponds to the up-down direction of the subject fixed to the forehead contact portion or the like. The Z direction corresponds to the depth direction of the apparatus main body and corresponds to the front-back direction of the subject fixed to the forehead contact portion or the like. In the examination of the eye to be examined E, it is necessary to move the apparatus main body so that the eye to be examined E is set at a predetermined position of the apparatus main body, and the alignment optical system is an optical system for obtaining a movement amount of the apparatus main body.

The alignment optical system includes an XY-direction position detection system and a Z-direction position detection system. The XY-direction position detection system is configured to detect a position in the XY directions (a shift in the up-down and left-right directions with respect to the apparatus main body) of the eye to be examined E (corneal vertex). The Z-direction position detection system is configured to detect a position in the front-back direction (a shift in the Z direction) of the eye to be examined E (corneal vertex).

The XY-direction position detection system includes an XY position detection light source 216, the hot mirror 213, the relay lens 214, the beam splitter 215, the beam splitter 206, the hot mirror 203, the objective lens 204, an image forming lens 217, and a two-dimensional position sensor 218. Alignment light for position detection emitted from the XY position detection light source 216 reaches the anterior segment Ec (cornea) of the eye to be examined E through the hot mirror 213, the relay lens 214, the beam splitter 215, the beam splitter 206, the hot mirror 203, and the objective lens 204.

Since the corneal surface of the eye to be examined E is formed in a spherical shape, the alignment light is reflected at the corneal surface so as to form a bright spot image inside the corneal vertex of the eye to be examined E. The reflected light reflected at the corneal surface is guided to the objective lens 204. The reflected light from the corneal vertex is input to the two-dimensional position sensor 218 through the objective lens 204, the hot mirror 203, the beam splitter 206, the beam splitter 215, and the image forming lens 217. The two-dimensional position sensor 218 detects the position of a bright spot as the position of the corneal vertex (the position in the X direction and the Y direction).

A detection signal of the two-dimensional position sensor 218 is input to the control unit 500. In the present embodiment, a position where the corneal vertex is to be disposed in a state where alignment between the two-dimensional position sensor 218 and the anterior segment photographing system is performed is set in advance as a normal position in the control unit 500. The normal position of the corneal vertex is a position to be followed at the time of tomographic image acquisition, and is, for example, the center position of a photographed image by an imaging element. The control unit 500 obtains a position shift amount in the X direction and the Y direction between the normal position and the detected corneal vertex (bright spot) on the basis of the detection by the two-dimensional position sensor 218.

The Z-direction position detection system includes a Z position detection light source 219, an image forming lens 220, and a line sensor 221. The Z position detection light source 219 irradiates the eye to be examined E with detection light (slit light or spot light) from the oblique direction. The irradiation light is reflected obliquely at the cornea, and the reflected light is guided to the line sensor 221 through the image forming lens 220. Since the reflected light incident on the line sensor 221 has a different incident position depending on the position in the front-back direction (Z direction) of the eye to be examined E, the Z-direction position of the eye to be examined E is detected.

The control unit 500 moves the apparatus main body with respect to the holding table so that the position shift amount in the X direction and the Y direction of the corneal vertex (bright spot) detected by the XY-direction position detection system and the position shift amount of the eye to be examined E detected by the Z-direction position detection system are all 0. As a result, the alignment is completed.

The k-clock generation interference optical system 400 illustrated in Fig. 1 is a device that optically generates a sample clock (k-clock) from input light branched from the SMFC 101 in order to sample the measurement interference signal at an equal interval frequency. A k-clock signal generated by the k-clock generation interference optical system 400 is input to the control unit 500. By the control unit 500 referring to the k-clock signal, distortion of the measurement interference signal is suppressed, and deterioration of resolution is prevented.

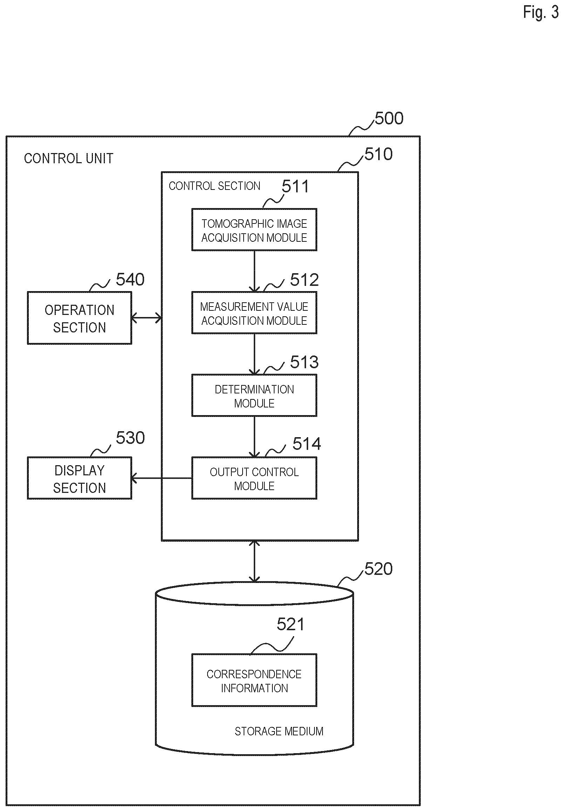

Fig. 3 is a configuration diagram of the control unit 500. The control unit 500 includes a control section 510, a storage medium 520, a display section 530, and an operation section 540. The control section 510 includes a processor, a ROM, and a RAM (not illustrated). The control section 510 performs various processes by executing a program stored in the storage medium 520. The control section 510 controls a control target included in each of the OCT interference system 100, the scanning-alignment optical system 200, the reference optical system 300, and the k-clock generation interference optical system 400, for example, a motor for alignment, the area sensor 208, and the like. The control section 510 also generates (acquires) a tomographic image on the basis of information output from the OCT interference system 100, the scanning-alignment optical system 200, and the like.

The storage medium 520 stores various programs and various data. The storage medium 520 stores correspondence information 521 indicating a correspondence between the biological age (BA) of the eye to be examined and the attribute value of a predetermined attribute of the eye to be examined. Hereinafter, this predetermined attribute is referred to as measurement target attribute. In the present embodiment, the measurement target attribute is the characteristic of a corner angle that is a portion in the anterior segment, and is a trabecular iris space area (TISA). The correspondence information 521 of the present embodiment is information indicating a correspondence relationship between the biological age and the attribute value of the sample 20, and in the present embodiment, is information in which information on the biological age and information on the range of the TISA value are associated with each other. The biological age is an index indicating how old of a biological tissue the biological tissue is statistically close to. Note that the age is an index indicating how long the subject has been born, and is a concept including not only the number of years elapsed from birth but also the age range (twenties, thirties, etc.) of the subject.

The TISA is the area of a predetermined region occurring in a plane parallel to incident light passing through the corneal vertex and including the corneal vertex. This predetermined region is a region surrounded by a line segment to the iris vertically drawn from a position at a predetermined distance (for example, 500 µm, 250 µm, and the like) from the scleral spur on the corneal posterior surface, a straight line drawn parallel to this line segment from the scleral spur, the corneal posterior surface, and the iris anterior surface. The length of this line segment (the line segment to the iris vertically drawn from the position at a predetermined distance from the scleral spur on the corneal posterior surface) is an angle opening distance (AOD). Hereinafter, the line segment to the iris vertically drawn from the position at a predetermined distance from the scleral spur on the corneal posterior surface is referred to as AOD line segment. Hereinafter, this predetermined distance is referred to as predetermined distance.

The correspondence information 521 is obtained by measuring the attribute value of the measurement target attribute for the eyes to be examined of a plurality of subjects of various ages in advance, and specifying which value range the attribute value of the measurement target attribute of the eye to be examined of each age is mainly in. The display section 530 displays various types of information. The display section 530 displays, for example, the tomographic image or the front image of the anterior segment. The operation section 540 receives an input from a user. The operation section 540 is, for example, a keyboard, a mouse, and the like. As another example, the operation section 540 may be a touch panel provided integrally with the display section 530.

The control section 510 functions as a tomographic image acquisition module 511, a measurement value acquisition module 512, a determination module 513, and an output control module 514 by executing a program stored in the storage medium 520. That is, in the following description, processing described as being performed by the tomographic image acquisition module 511, the measurement value acquisition module 512, the determination module 513, and the output control module 514 is processing executed by the control section 510 (processor).

The tomographic image acquisition module 511 controls the OCT interference system 100, the scanning-alignment optical system 200 (such as the galvano scanner 202), and the k-clock generation interference optical system 400 to acquire a measurement interference signal. Then, the tomographic image acquisition module 511 performs processing such as inverse Fourier transform on the measurement interference signal to acquire a tomographic image of the anterior segment Ec along a scanning line.

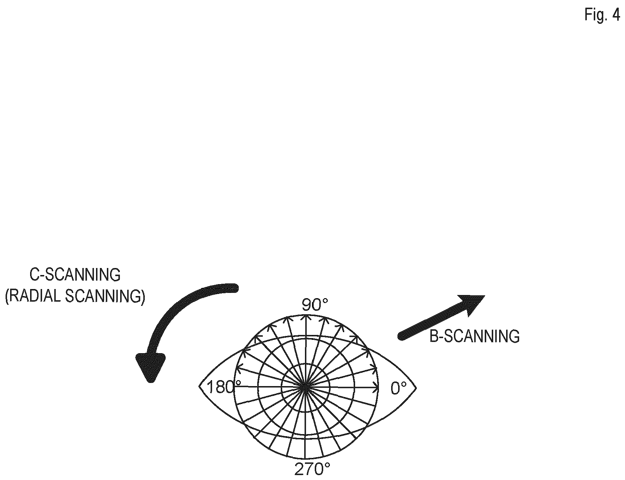

The tomographic image acquisition module 511 changes a scanning direction in the galvano scanner 202 and acquires tomographic images of a plurality of slice planes. In the present embodiment, the tomographic image acquisition module 511 performs radial scanning illustrated in Fig. 4. In the radial scanning, a B-scanning direction is the radiation direction centered on the corneal vertex of the eye to be examined E, a C-scanning direction is the circumferential direction of the anterior segment surface of the eye to be examined E, and a two-dimensional tomographic image of each slice surface is captured. Note that the center of the radial scanning may not strictly coincide with the corneal vertex. As another example, the center of the radial scanning may be a position that substantially coincides with the corneal vertex, such as the pupil center or the corneal diameter center. Each tomographic image thus obtained includes the corner angle of the anterior segment Ec at two positions. A plurality of tomographic images constituting a three-dimensional image of the anterior segment of the eye to be examined E are obtained by the radial scanning. In the present embodiment, the plurality of tomographic images are acquired at predetermined angular intervals. In the present embodiment, the angular interval is 10°, but may be another angle such as 1° or 15° as another example. Therefore, 18 tomographic images are acquired at 10° intervals. In this manner, the tomographic image acquisition module 511 acquires the plurality of tomographic images including the corner angle in different radiation directions centered on the corneal vertex.

In the present embodiment, a measurement position, which is the position of the tomographic image in the radial scanning where the corner angle is obtained, is represented by a rotation angle from a predetermined position of a circle centered on the corneal vertex. In the present embodiment, the position on the right side of the anterior segment in a front image is referred to as a rotation angle of 0°, the upper side is referred to as a rotation angle of 90°, the left side is referred to as a rotation angle of 180°, and the lower side is referred to as a rotation angle of 270°. In the present embodiment, the measurement position takes a value of 0° to 170° in increments of 10°.

The measurement value acquisition module 512 measures the attribute value of the measurement target attribute of the sample 20 that is the eye to be examined to acquire a measurement value on the basis of the tomographic images acquired by the tomographic image acquisition module 511.

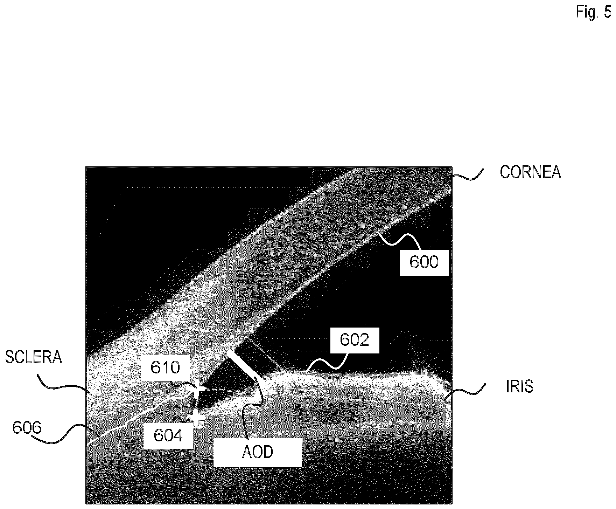

The measurement value acquisition module 512 obtains the TISA value from each of the plurality of tomographic images. In Fig. 5, the AOD line segment is indicated by a white line. Note that Fig. 5 is a drawing illustrating a tomographic image showing a portion around the corner angle.

It can be understood that the smaller the AOD value, the narrower the corner angle. Diseases with a narrower corner angle include primary angle closure suspect (PACS), primary angle closure (PAC), primary angle closure glaucoma (PACG), acute glaucoma attack, acute primary angle closure glaucoma, and secondary glaucoma (direct angle closure due to 1. peripheral anterior synechiae occurring independently of the anterior chamber depth, 2. anterior movement of the iris-lens by a cause other than pupillary block). For example, the primary angle closure is a state with a narrow corner angle. The angle closure glaucoma is a state with a narrow corner angle, increased outflow resistance of aqueous humor, and increased intraocular pressure to be causing a damage to the optic nerve. The acute glaucoma attack is a state in which the corner angle and the iris are blocked, the aqueous humor cannot flow out, and the intraocular pressure rapidly rises.

The measurement value acquisition module 512 acquires the measurement value of the TISA by performing the following processing on each of the plurality of tomographic images.

The tomographic image includes the two corner angles: a corner angle in the direction of the measurement position centered on the corneal vertex and a corner angle in the direction of +180° with respect to the measurement position centered on the corneal vertex. Therefore, the measurement value acquisition module 512 performs the following processing on each of the two corner angles of the tomographic image. The measurement value acquisition module 512 specifies the position of the scleral spur from an image around the corner angle of the tomographic image. Hereinafter, the position of the scleral spur is referred to as SS position. Specifically, the measurement value acquisition module 512 first extracts the image around the corner angle from the tomographic image. As a result, for example, an image as illustrated in Fig. 5 is extracted. Hereinafter, the extracted image is referred to as local image. The measurement value acquisition module 512 detects a corneal posterior surface boundary line 600 and an iris anterior surface boundary line 602 on the basis of a luminance gradient in the local image. The corneal posterior surface boundary line 600 is a boundary line corresponding to the surface of the cornea on the iris side. The iris anterior surface boundary line 602 is a boundary line corresponding to the surface of the iris on the cornea side. These boundary lines are detected by comparison with a threshold value of the luminance gradient. In addition, the boundary lines may be detected using a boundary line detection filter.

The measurement value acquisition module 512 specifies a sclera-uvea boundary line 606 on the basis of the magnitude of the luminance gradient with reference to a curved point (corresponding to an angle recess 604) of a boundary line formed by connecting the corneal posterior surface boundary line 600 and the iris anterior surface boundary line 602. Here, the sclera-uvea boundary line 606 is a boundary line corresponding to a boundary between the sclera and the uvea.

Then, the measurement value acquisition module 512 specifies an intersection among the sclera-uvea boundary line 606, the corneal posterior surface boundary line 600, and the iris anterior surface boundary line 602 as an SS position 610. The measurement value acquisition module 512 may use the technique described in, for example, JP 2015-66084 A as a process of specifying the SS position 610.

Note that the measurement value acquisition module 512 only needs to specify the SS position on the basis of the tomographic image, and specific processing therefor is not limited to that in the present embodiment. As another example, the measurement value acquisition module 512 may specify the SS position using an SS position estimation model. In this case, it is assumed that the SS position estimation model is learned (generated) using a correct image in which the SS position is specified for testing, and the SS position estimation model is stored in the storage medium 520. As yet another example, the tomographic image may be displayed on the display section 530, and the SS position may be specified by the user.

The measurement value acquisition module 512 specifies a position at a predetermined distance from the SS position 610 in the direction of the corneal vertex on the corneal posterior surface boundary line 600. Then, the measurement value acquisition module 512 specifies an intersection between a line perpendicular to the corneal posterior surface boundary line 600 extending from the specified position and the iris anterior surface boundary line 602. The measurement value acquisition module 512 acquires, as the AOD, the distance of a line segment (AOD line segment) connecting the specified position and the specified intersection. Then, the measurement value acquisition module 512 specifies a region surrounded by a straight line passing through the SS position 610 and parallel to the AOD line segment, the corneal posterior surface boundary line 600, the iris anterior surface boundary line 602, and the AOD line segment, and acquires the area of the specified region as the TISA.

Through the above processing, the measurement value acquisition module 512 acquires the TISA in each direction centered on the corneal vertex.

The determination module 513 determines, as the estimated age of the sample 20, the biological age of the sample 20 that is the eye to be examined on the basis of the measurement value of the TISA acquired by the measurement value acquisition module 512 and the correspondence information 521. In the present embodiment, the correspondence information 521 indicates correspondence information between the range of the TISA value and the age for each direction centered on the corneal vertex.

The determination module 513 specifies a biological age corresponding to the measurement value from the correspondence information 521 for each of the measurement values of the TISA in the respective directions acquired by the measurement value acquisition module 512. Then, the determination module 513 determines, as the estimated age of the sample 20, the average of the biological ages specified for the measurement values of the TISA in the respective directions. However, as another example, the determination module 513 may determine, as the estimated age of the sample 20, another value such as another statistical value (a median, a mode, etc.) for the biological ages specified for the measurement values of the TISA in the respective directions.

The output control module 514 outputs evaluation information of the sample 20 on the basis of the estimated age of the sample 20 determined by the determination module 513 and the actual age of the sample 20 (the actual age of the subject). In the present embodiment, the actual age of the sample 20 is specified in advance and stored in the storage medium 520. In the present embodiment, in a case where a difference value obtained by subtracting the actual age of the sample 20 from the estimated age of the sample 20 is equal to or more than a predetermined age threshold value (for example, 5, 10, and the like), the output control module 514 generates, as the evaluation information, information indicating that there is a possibility that some sort of abnormality (for example, disease, aging, and the like) has occurred in the measurement target attribute of the sample 20. In the present embodiment, the output control module 514 generates, as the evaluation information, information indicating that there is a possibility that an abnormality has occurred in the corner angle of the sample 20. In the present embodiment, the output control module 514 generates, as the evaluation information, text information (for example, "pay attention to the corner angle" or the like) indicating that there is a possibility that an abnormality has occurred in the corner angle of the sample 20. Then, the output control module 514 outputs the generated evaluation information by causing the display section 530 to display the evaluation information. However, as another example, the output control module 514 may output the generated evaluation information by voice using a speaker, may output the generated evaluation information by printing the evaluation information on a print medium such as paper, or may output the generated evaluation information by transmitting the evaluation information to a predetermined transmission destination.

As described above, with the configuration of the present embodiment, the estimation system 1 can determine the estimated age of the sample 20 that is the eye to be examined from the measurement value of the measurement target attribute of the eye to be examined and the correspondence information 521.

The estimation system 1 outputs the evaluation information of the sample 20 on the basis of the estimated age of the sample 20 and the actual age of the sample 20. As a result, the estimation system 1 can present the state of the sample 20 to the user. In addition, when the value obtained by subtracting the actual age of the sample 20 from the estimated age of the sample 20 is equal to or more than the predetermined age threshold value, the estimation system 1 outputs the evaluation information indicating that there is a possibility that an abnormality has occurred in the corner angle of the eye to be examined. As a result, the estimation system 1 can present the possibility of an abnormality in the corner angle of the sample 20 to the user.

### (1-2) Estimation processing:

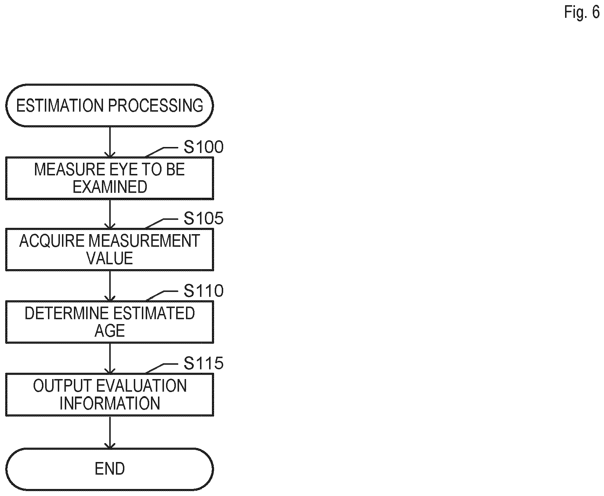

Fig. 6 is a flowchart illustrating estimation processing by the control section 510. The estimation processing is a process of estimating the age of the sample 20 that is the eye to be examined. In the estimation processing, the tomographic images are acquired, and it is assumed that the alignment in the scanning-alignment optical system is completed before the acquisition.

In step S100, the tomographic image acquisition module 511 acquires a plurality of tomographic images constituting a three-dimensional image. Specifically, the tomographic image acquisition module 511 acquires a plurality of tomographic images constituting a three-dimensional image of the anterior segment while changing the scanning direction in the galvano scanner 202. In the present embodiment, 18 tomographic images are obtained by the radial scanning at intervals of 10°. After the tomographic image acquisition module 511 completes the processing in step S100, the processing proceeds to step S105.

In step S105, the measurement value acquisition module 512 acquires the TISA value for each of the two corner angles included in each tomographic image obtained in step S100 as the measurement value. After the measurement value acquisition module 512 completes the processing in step S105, the processing proceeds to step S110. Step S105 is an example of an acquisition step.

In step S110, the determination module 513 determines the biological age of the sample 20 that is the eye to be examined as the estimated age of the sample 20 on the basis of the measurement value of the TISA acquired in step S105 and the correspondence information 521. After the determination module 513 completes the processing in step S110, the processing proceeds to step S115. Step S115 is an example of a determination step.

In step S115, the output control module 514 outputs the evaluation information of the sample 20 on the basis of the estimated age of the sample 20 determined by the determination module 513 and the actual age of the sample 20. In the present embodiment, in a case where the difference value obtained by subtracting the actual age of the sample 20 from the estimated age of the sample 20 is equal to or more than the predetermined age threshold value, the output control module 514 generates, as the evaluation information, information indicating that there is a possibility that some sort of abnormality has occurred in the measurement target attribute of the sample 20. Then, the output control module 514 outputs the generated evaluation information by causing the display section 530 to display the evaluation information.

### (2) Second embodiment:

As a second embodiment, an embodiment in a case where the measurement target attribute is the characteristic of the cornea that is a portion in the anterior segment of the sample 20 that is the eye to be examined will be described. In the present embodiment, the measurement target attribute is an astigmatism axis angle. As the eyeball ages, the astigmatism axis is inclined, and the eyeball tends to have inverse astigmatism.

The estimation system 1 of the present embodiment has a hardware configuration similar to that of the first embodiment.

The correspondence information 521 of the present embodiment is information in which information on the value of the astigmatism axis angle and information on the biological age are associated with each other.

Similarly to the first embodiment, the control section 510 functions as the tomographic image acquisition module 511, the measurement value acquisition module 512, the determination module 513, and the output control module 514 by executing a program stored in the storage medium 520. That is, in the following description, processing described as being performed by the tomographic image acquisition module 511, the measurement value acquisition module 512, the determination module 513, and the output control module 514 is processing executed by the control section 510 (processor). Hereinafter, the processing of each of the tomographic image acquisition module 511, the measurement value acquisition module 512, the determination module 513, and the output control module 514 in the present embodiment will be described.

The tomographic image acquisition module 511 controls the OCT interference system 100, the scanning-alignment optical system 200 (such as the galvano scanner 202), and the k-clock generation interference optical system 400 to acquire a measurement interference signal. Then, the tomographic image acquisition module 511 performs processing such as inverse Fourier transform on the measurement interference signal to acquire a tomographic image of the anterior segment Ec along a scanning line.

The tomographic image acquisition module 511 changes a scanning direction in the galvano scanner 202 and acquires tomographic images of a plurality of slice planes. In the present embodiment, the tomographic image acquisition module 511 performs radial scanning similarly to the first embodiment. A plurality of tomographic images constituting a three-dimensional image of the anterior segment of the eye to be examined E are obtained by the radial scanning. In this manner, the tomographic image acquisition module 511 acquires a plurality of tomographic images in different radiation directions centered on the corneal vertex.

The measurement value acquisition module 512 measures the attribute value of the measurement target attribute of the sample 20 that is the eye to be examined to acquire a measurement value on the basis of the tomographic images acquired by the tomographic image acquisition module 511.

The measurement value acquisition module 512 performs the following processing on each of the plurality of tomographic images. The measurement value acquisition module 512 detects the boundary line of the corneal anterior surface from the tomographic image. Then, the measurement value acquisition module 512 derives curvature radii at a plurality of positions on the detected boundary line. The measurement value acquisition module 512 obtains refractive power at each position on the boundary line as the refractive power of the cornea at this position on the basis of the derived curvature radii. More specifically, at each position on the boundary line, the measurement value acquisition module 512 obtains the refractive power of the cornea at this position by multiplying the reciprocal of the curvature radius at this position by a predetermined constant.

The measurement value acquisition module 512 acquires the refractive power at a plurality of positions on the cornea at the respective measurement positions by the above processing.

The measurement value acquisition module 512 specifies the distribution of the refractive power on the cornea on the basis of the acquired refractive power at the plurality of positions on the cornea. Then, the measurement value acquisition module 512 makes the specified distribution approximate to an elliptical sphere using a known method such as a least squares method. The measurement value acquisition module 512 specifies the major axis of the approximate elliptical sphere as a steep meridian. In addition, the measurement value acquisition module 512 specifies a line perpendicular to incident light and perpendicular to the specified steep meridian as a flat meridian.

The measurement value acquisition module 512 specifies the inclination of the specified steep meridian when the sample 20 is viewed from the front, as the astigmatism axis angle of the sample 20. The measurement value acquisition module 512 acquires the specified astigmatism axis angle as the measurement value of the measurement target attribute of the sample 20.

The determination module 513 determines an estimated age for the cornea of the sample 20 that is the eye to be examined on the basis of the measurement value of the astigmatism axis angle acquired by the measurement value acquisition module 512 and the correspondence information 521. More specifically, the determination module 513 acquires a biological age corresponding to the measurement value of the astigmatism axis angle from the correspondence information 521, and determines the acquired biological age as the estimated age for the cornea of the sample 20.

The output control module 514 outputs evaluation information of the sample 20 on the basis of the estimated age of the sample 20 determined by the determination module 513 and the actual age of the sample 20. In the present embodiment, the actual age of the sample 20 is specified in advance and stored in the storage medium 520.

In a case where a difference value obtained by subtracting the actual age from the estimated age of the sample 20 is equal to or more than a predetermined age threshold value (for example, 5, 10, and the like), the output control module 514 generates, as the evaluation information, information indicating that there is a possibility of an abnormality in the cornea of the sample 20. Presbyopia and cataract eyeballs tend to have inverse astigmatism. Therefore, in the present embodiment, the output control module 514 generates information indicating that there is a possibility of presbyopia or cataract in the sample 20 as the information indicating that there is a possibility of an abnormality in the cornea of the sample 20. In the present embodiment, the output control module 514 generates, as the evaluation information, text information (for example, "pay attention to presbyopia/cataract" or the like) indicating that there is a possibility of presbyopia or cataract in the sample 20. However, as another example, the output control module 514 may generate, as the evaluation information, text information (for example, "pay attention to the cornea" or the like) indicating that there is a possibility of an abnormality in the cornea of the sample 20. Then, the output control module 514 outputs the generated evaluation information by causing the display section 530 to display the evaluation information. However, as another example, the output control module 514 may output the generated evaluation information by voice using a speaker, may output the generated evaluation information by printing the evaluation information on a print medium such as paper, or may output the generated evaluation information by transmitting the evaluation information to a predetermined transmission destination.

As described above, with the configuration of the present embodiment, the estimation system 1 can present the possibility of an abnormality in the cornea of the sample 20 to the user.

### (3) Third embodiment:

As a third embodiment, an embodiment in a case where the measurement target attribute is the characteristic of the lens that is a portion in the anterior segment of the sample 20 that is the eye to be examined will be described. In the present embodiment, the measurement target attribute is the luminance distribution of the lens nucleus, the thickness of the lens, and the equatorial diameter of the lens.

The estimation system 1 of the present embodiment has a hardware configuration similar to that of the first embodiment.

The correspondence information 521 of the present embodiment includes information in which information on the value of the luminance distribution of the lens nucleus and information on the biological age are associated with each other, information in which information on the value of the thickness of the lens and information on the biological age are associated with each other, and information in which information on the value of the equatorial diameter of the lens and information on the biological age are associated with each other.

Similarly to the first embodiment, the control section 510 functions as the tomographic image acquisition module 511, the measurement value acquisition module 512, the determination module 513, and the output control module 514 by executing a program stored in the storage medium 520. That is, in the following description, processing described as being performed by the tomographic image acquisition module 511, the measurement value acquisition module 512, the determination module 513, and the output control module 514 is processing executed by the control section 510 (processor). Hereinafter, the processing of each of the tomographic image acquisition module 511, the measurement value acquisition module 512, the determination module 513, and the output control module 514 in the present embodiment will be described.

The tomographic image acquisition module 511 controls the OCT interference system 100, the scanning-alignment optical system 200 (such as the galvano scanner 202), and the k-clock generation interference optical system 400 to acquire a measurement interference signal. Then, the tomographic image acquisition module 511 performs processing such as inverse Fourier transform on the measurement interference signal to acquire a tomographic image of the anterior segment Ec along a scanning line.

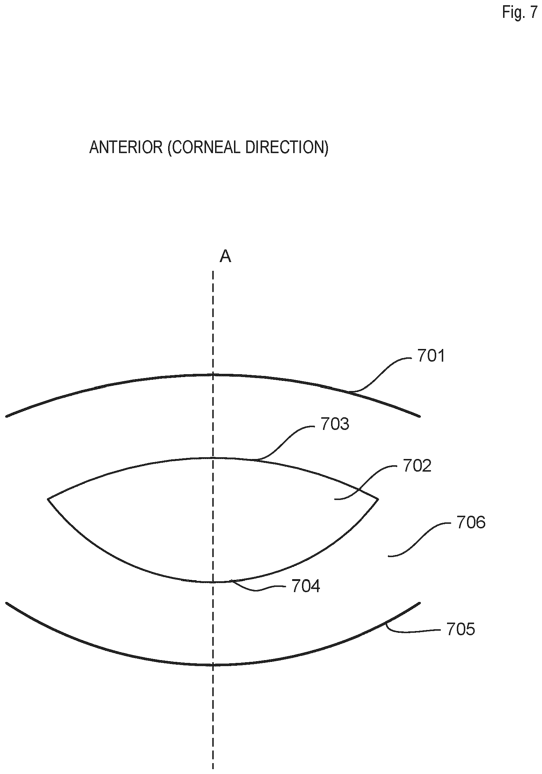

The tomographic image acquisition module 511 acquires a tomographic image around the lens of the sample 20 that is the eye to be examined by measuring the sample 20. Fig. 7 illustrates a schematic view of the image around the lens acquired here.

The measurement value acquisition module 512 detects an anterior surface 701 of the lens, an anterior surface 703 of a lens nucleus 702, a posterior surface 704 of the lens nucleus 702, and a posterior surface 705 of the lens from the tomographic image around the lens acquired by the tomographic image acquisition module 511 by detecting the boundary line. Note that the lens nucleus 702 is surrounded by a lens cortex 706. The measurement value acquisition module 512 specifies the positions of the detected anterior surface 701 and the detected posterior surface 705 of the lens, and acquires a distance between the specified positions of the anterior surface 701 and the posterior surface 705 as the measurement value of the thickness of the lens.

In addition, the measurement value acquisition module 512 specifies the distribution of luminance values in the image of the lens nucleus along a straight line (straight line A in Fig. 7) parallel to incident light passing through the corneal vertex. The measurement value acquisition module 512 acquires the specified distribution of the luminance values as the measurement value of the distribution of the luminance values of the lens nucleus. When the lens nucleus 702 becomes cloudy due to cataract or the like, the luminance value in the lens nucleus 702 increases. Therefore, the luminance distribution of the lens nucleus 702 is a value reflecting the state of the sample 20 that is the eye to be examined.

In addition, the measurement value acquisition module 512 acquires the measurement value of the diameter of the equatorial part (hereinafter, the equatorial diameter) of the lens as described below. The equatorial diameter of the lens is obtained as a distance between two intersecting points of the curve of the lens anterior surface and the curve of the lens posterior surface. The measurement value acquisition module 512 obtains an approximate curve in the cross section of the anterior surface 701 of the lens detected from the tomographic image of the cross section of the eye to be examined. Hereinafter, this approximate curve is referred to as anterior-surface approximate curve. In addition, the measurement value acquisition module 512 obtains an approximate curve in the cross section of the posterior surface 705 of the lens detected from the tomographic image. Hereinafter, this approximate curve is referred to as posterior-surface approximate curve. Then, the measurement value acquisition module 512 specifies two intersections between the anterior-surface approximate curve and the posterior-surface approximate curve, and acquires a distance between the specified two intersections as the measurement value of the equatorial diameter of the lens of the sample 20 that is the eye to be examined.

The determination module 513 determines an estimated age for the distribution of the luminance values of the lens nucleus 702 of the sample 20 that is the eye to be examined on the basis of the measurement value of the distribution of the luminance values of the lens nucleus 702 acquired by the measurement value acquisition module 512 and the correspondence information 521. More specifically, the determination module 513 obtains similarity between the distribution of the luminance values as the measurement value and each distribution of the luminance values indicated by the correspondence information 521. In the present embodiment, the determination module 513 uses the magnitude of the inner product of vectors when each distribution is regarded as a vector as the similarity between the distributions of the luminance values. However, the determination module 513 may use another value as the similarity between the distributions of the luminance values. For example, the determination module 513 may obtain a correlation value between the distribution of the luminance values as the measurement value and each distribution of the luminance values indicated by the correspondence information 521 using an image correlation method, and use the obtained correlation value as the similarity between these distributions. The determination module 513 specifies a distribution having the largest similarity to the measurement value among the distributions of the luminance values indicated by the correspondence information 521. The determination module 513 acquires a biological age corresponding to the specified distribution from the correspondence information 521. The determination module 513 determines the acquired biological age as the estimated age of the sample 20 for the distribution of the luminance values of the lens nucleus 702. Hereinafter, the estimated age of the sample 20 for the distribution of the luminance values of the lens nucleus 702 is referred to as luminance value estimated age.

In addition, the determination module 513 determines an estimated age for the thickness of the lens of the sample 20 that is the eye to be examined on the basis of the measurement value of the thickness of the lens acquired by the measurement value acquisition module 512 and the correspondence information 521. More specifically, the determination module 513 acquires a biological age corresponding to the measurement value of the thickness of the lens from the correspondence information 521. The determination module 513 determines the acquired biological age as the estimated age of the sample 20 for the thickness of the lens. Hereinafter, the estimated age of the sample 20 for the thickness of the lens is referred to as thickness estimated age.

In addition, the determination module 513 determines an estimated age for the equatorial diameter of the lens of the sample 20 that is the eye to be examined on the basis of the measurement value of the equatorial diameter of the lens acquired by the measurement value acquisition module 512 and the correspondence information 521. More specifically, the determination module 513 acquires a biological age corresponding to the measurement value of the equatorial diameter of the lens from the correspondence information 521. The determination module 513 determines the acquired biological age as the estimated age of the sample 20 for the equatorial diameter of the lens. Hereinafter, the estimated age of the sample 20 for the equatorial diameter of the lens is referred to as equatorial diameter estimated age.

In the present embodiment, the determination module 513 obtains the estimated age of the sample 20 in consideration of the race of the subject and the gender of the subject as well. In the present embodiment, a correction value for age (for example, a value that becomes larger in a race in which the state of the lens tends to deteriorate, or the like) is determined with respect to each race by investigating the influence of a race difference on the health condition of the lens in advance. Hereinafter, the correction value for age with respect to race is referred to as race correction value.

In addition, a correction value for age (for example, a value that becomes larger in a gender that tends to have poor eyesight, or the like) is determined with respect to each gender by investigating the influence of a gender difference on eyesight in advance. Hereinafter, the correction value for age with respect to gender is referred to as gender correction value.

The determination module 513 determines a final estimated age for the lens of the sample 20 on the basis of the luminance value estimated age, the thickness estimated age, the equatorial diameter estimated age, the actual age of the subject, the race correction value corresponding to the race of the subject, and the gender correction value corresponding to the gender of the subject. In the present embodiment, the actual age of the sample 20 is specified in advance and stored in the storage medium 520.

In the present embodiment, the determination module 513 determines the final estimated age by weighted-averaging each of the luminance value estimated age, the thickness estimated age, the equatorial diameter estimated age, the actual age of the subject, the race correction value corresponding to the race of the subject, and the gender correction value corresponding to the gender of the subject with a predetermined weight. This weight is determined in advance according to the degree of contribution to the eyesight of each of the distribution of the luminance values of the lens nucleus 702, the thickness of the lens, the equatorial diameter of the lens, the actual age of the subject, the race of the subject, and the gender of the subject. However, this weight may be another value (for example, an equal value for all the elements, or the like).

The output control module 514 outputs evaluation information of the sample 20 on the basis of the estimated age for the lens of the sample 20 determined by the determination module 513 and the actual age of the sample 20.

In a case where a difference value obtained by subtracting the actual age from the estimated age for the lens of the sample 20 is equal to or more than a predetermined age threshold value (for example, 5, 10, and the like), the output control module 514 generates, as the evaluation information, information indicating that there is a possibility of an abnormality in the lens of the sample 20. As the lens ages, the possibility of cataract also increases. Therefore, in the present embodiment, the output control module 514 generates information indicating that there is a possibility of cataract in the sample 20 as the information indicating that there is a possibility of an abnormality in the lens of the sample 20. In the present embodiment, the output control module 514 generates, as the evaluation information, text information (for example, "pay attention to cataract" or the like) indicating that there is a possibility of cataract in the sample 20. However, as another example, the output control module 514 may generate, as the evaluation information, text information (for example, "pay attention to the lens" or the like) indicating that there is a possibility of an abnormality in the lens of the sample 20. The output control module 514 outputs the generated evaluation information by causing the display section 530 to display the evaluation information. However, as another example, the output control module 514 may output the generated evaluation information by voice using a speaker, may output the generated evaluation information by printing the evaluation information on a print medium such as paper, or may output the generated evaluation information by transmitting the evaluation information to a predetermined transmission destination.

As described above, with the configuration of the present embodiment, the estimation system 1 can present the possibility of an abnormality in the lens of the sample 20 to the user. In addition, the estimation system 1 can present that there is a possibility of cataract in the sample 20 to the user.

### (4) Fourth embodiment:

As a fourth embodiment, an embodiment in a case where the measurement target attribute is the dimension value of the sample 20 that is the eye to be examined, and the characteristic of the anterior chamber/lens that is a portion in the anterior segment will be described. In the present embodiment, the measurement target attribute is the eye axis length, the thickness of the lens, and the anterior chamber depth.

The estimation system 1 of the present embodiment has a hardware configuration similar to that of the first embodiment.

The correspondence information 521 of the present embodiment includes information in which information on the value of the eye axis length and information on the biological age are associated with each other, information in which information on the value of the anterior chamber depth and information on the biological age are associated with each other, and information in which information on the value of the thickness of the lens and information on the biological age are associated with each other.

Similarly to the first embodiment, the control section 510 functions as the tomographic image acquisition module 511, the measurement value acquisition module 512, the determination module 513, and the output control module 514 by executing a program stored in the storage medium 520. That is, in the following description, processing described as being performed by the tomographic image acquisition module 511, the measurement value acquisition module 512, the determination module 513, and the output control module 514 is processing executed by the control section 510 (processor). Hereinafter, the processing of each of the tomographic image acquisition module 511, the measurement value acquisition module 512, the determination module 513, and the output control module 514 in the present embodiment will be described.

The tomographic image acquisition module 511 controls the OCT interference system 100, the scanning-alignment optical system 200 (such as the galvano scanner 202), and the k-clock generation interference optical system 400 to acquire a measurement interference signal. Then, the tomographic image acquisition module 511 performs processing such as inverse Fourier transform on the measurement interference signal to acquire a tomographic image of the anterior segment Ec along a scanning line.

The tomographic image acquisition module 511 measures the sample 20 while adjusting the position of the reference section 301 of the reference optical system 300, thereby acquiring a tomographic image around the cornea and the lens of the sample 20 that is the eye to be examined and a tomographic image around the retina.

The measurement value acquisition module 512 detects the corneal vertex from the tomographic image around the cornea and the lens acquired by the tomographic image acquisition module 511 and specifies the position of the detected corneal vertex. In addition, the measurement value acquisition module 512 detects an intersection between incident light passing through the corneal vertex and the retina from the tomographic image around the retina acquired by the tomographic image acquisition module 511, and specifies the position of the detected intersection. The measurement value acquisition module 512 acquires a distance between the specified position of the corneal vertex and the position of the retina as the measurement value of the eye axis length.

In addition, the measurement value acquisition module 512 detects an intersection between the incident light passing through the corneal vertex and the corneal posterior surface from the tomographic image around the cornea and the lens acquired by the tomographic image acquisition module 511, and specifies the position of the detected intersection. In addition, the measurement value acquisition module 512 detects an intersection between the incident light passing through the corneal vertex and the lens anterior surface from the tomographic image around the cornea and the lens acquired by the tomographic image acquisition module 511, and specifies the position of the detected intersection. Then, the measurement value acquisition module 512 acquires a distance between the position of the intersection between the incident light passing through the corneal vertex and the corneal posterior surface and the position of the intersection between the incident light passing through the corneal vertex and the lens anterior surface as the measurement value of the anterior chamber depth.

In addition, the measurement value acquisition module 512 acquires the measurement value of the thickness of the lens from the tomographic image around the lens acquired by the tomographic image acquisition module 511 by the same method as in the above-described third embodiment.

The determination module 513 determines an estimated age for the eye axis length of the sample 20 that is the eye to be examined on the basis of the measurement value of the eye axis length acquired by the measurement value acquisition module 512 and the correspondence information 521. More specifically, the determination module 513 acquires a biological age corresponding to the measurement value of the eye axis length from the correspondence information 521, and determines the acquired biological age as the estimated age for the eye axis length of the sample 20. Hereinafter, the estimated age for the eye axis length of the sample 20 is referred to as eye axis length estimated age.

The determination module 513 determines an estimated age for the anterior chamber depth of the sample 20 that is the eye to be examined on the basis of the measurement value of the anterior chamber depth acquired by the measurement value acquisition module 512 and the correspondence information 521. More specifically, the determination module 513 the determination module 513 acquires a biological age corresponding to the measurement value of the anterior chamber depth from the correspondence information 521, and determines the acquired biological age as the estimated age for the anterior chamber depth of the sample 20. Hereinafter, the estimated age for the anterior chamber depth of the sample 20 is referred to as anterior chamber depth estimated age.

The determination module 513 determines an estimated age for the thickness of the lens of the sample 20 that is the eye to be examined on the basis of the measurement value of the thickness of the lens acquired by the measurement value acquisition module 512 and the correspondence information 521. More specifically, the determination module 513 the determination module 513 acquires a biological age corresponding to the measurement value of the thickness of the lens from the correspondence information 521, and determines the acquired biological age as the estimated age (thickness estimated age) for the thickness of the lens of the sample 20.

The output control module 514 outputs evaluation information of the sample 20 on the basis of the estimated age of the sample 20 determined by the determination module 513 and the actual age of the sample 20. In the present embodiment, the actual age of the sample 20 is specified in advance and stored in the storage medium 520.

In a case where a difference value obtained by subtracting the actual age from the eye axis length estimated age of the sample 20 is equal to or more than a predetermined age threshold value (for example, 5, 10, and the like), the output control module 514 generates, as the evaluation information, information indicating that there is a possibility of an abnormality in the eye axis length of the sample 20. In the present embodiment, the output control module 514 generates, as the evaluation information, text information (for example, "pay attention to the eye axis length" or the like) indicating that there is a possibility of an abnormality in the eye axis length of the sample 20.

In addition, in a case where a difference value obtained by subtracting the actual age from the anterior chamber depth estimated age of the sample 20 is equal to or more than a predetermined age threshold value (for example, 5, 10, and the like), the output control module 514 generates, as the evaluation information, information indicating that there is a possibility of an abnormality in the anterior chamber depth of the sample 20. In the present embodiment, the output control module 514 generates, as the evaluation information, text information (for example, "pay attention to the anterior chamber depth" or the like) indicating that there is a possibility of an abnormality in the anterior chamber depth of the sample 20.

In addition, in a case where a difference value obtained by subtracting the actual age from the thickness estimated age of the sample 20 is equal to or more than a predetermined age threshold value (for example, 5, 10, and the like), the output control module 514 generates, as the evaluation information, information indicating that there is a possibility of an abnormality in the thickness of the lens of the sample 20. In the present embodiment, the output control module 514 generates, as the evaluation information, text information (for example, "pay attention to the thickness of the lens" or the like) indicating that there is a possibility of an abnormality in the thickness of the lens of the sample 20.

Note that the respective age threshold values compared with the difference value obtained by subtracting the actual age from the eye axis length estimated age, the difference value obtained by subtracting the actual age from the anterior chamber depth estimated age, and the difference value obtained by subtracting the actual age from the thickness estimated age may be the same value or different values.

The eye axis length, the anterior chamber depth, and the thickness of the lens tend to vary with aging. Specifically, the thickness of the lens tends to increase. In addition, as a result of increase in the thickness of the lens, the anterior chamber depth becomes shorter. Thus, the anterior chamber depth tends to become shorter. In this manner, the thickness of the lens and the anterior chamber depth have a negative correlation. In addition, the eye axis length tends to become shorter. As the eye axis length and the anterior chamber depth become shorter, presbyopia tends to increase. As the thickness of the lens increases, presbyopia tends to increase. As the eye axis length becomes longer, myopia tends to increase. Therefore, in the present embodiment, in a case where the difference value obtained by subtracting the actual age from the eye axis length estimated age of the sample 20 is equal to or more than the predetermined age threshold value, the difference value obtained by subtracting the actual age from the thickness estimated age of the sample 20 is equal to or more than the predetermined age threshold value, and the difference value obtained by subtracting the actual age from the anterior chamber depth estimated age of the sample 20 is equal to or more than the predetermined age threshold value, the output control module 514 further generates information indicating that there is a possibility of presbyopia in the sample 20 as the evaluation information. In the present embodiment, the output control module 514 generates, as the evaluation information, text information (for example, "pay attention to presbyopia" or the like) indicating that there is a possibility of presbyopia in the sample 20.

Then, the output control module 514 outputs the generated evaluation information by causing the display section 530 to display the evaluation information. However, as another example, the output control module 514 may output the generated evaluation information by voice using a speaker, may output the generated evaluation information by printing the evaluation information on a print medium such as paper, or may output the generated evaluation information by transmitting the evaluation information to a predetermined transmission destination.

As described above, with the configuration of the present embodiment, the estimation system 1 can present the possibility of an abnormality in the eye axis length, the anterior chamber depth, or the thickness of the lens of the sample 20 to the user. In addition, the estimation system 1 can present that there is a possibility of presbyopia in the sample 20 to the user.

### (5) Other embodiments:

The above embodiments are merely examples for carrying out the present invention, and various other embodiments can be adopted. Therefore, at least some of the configurations of the above-described embodiments may be omitted or replaced.

In the first embodiment described above, the measurement target attribute is the characteristic of the corner angle and is the TISA. However, as another example, the measurement target attribute may be another characteristic of the corner angle such as an AOD, an angle recess area (ARA), and a trabecular iris angle (TIA). For example, in a case where the AOD is used as the measurement target attribute, the correspondence information 521 indicates a correspondence between the AOD and the biological age. The measurement value acquisition module 512 acquires the AOD at each measurement position of the sample 20. Then, the determination module 513 may determine the estimated age of the sample 20 on the basis of the AOD acquired by the measurement value acquisition module 512 and the correspondence information 521. In addition, the measurement target attribute may be a plurality of characteristics of the corner angle.

In the second embodiment described above, the measurement target attribute is the characteristic of the cornea and is the astigmatism axis angle. However, the measurement target attribute may be another characteristic of the cornea. For example, the measurement target attribute may be the thickness of the cornea. In addition, it is known that the number of corneal endothelial cells decreases with aging. Therefore, the number of corneal endothelial cells can be regarded as an index reflecting age. Therefore, the measurement target attribute may be the number of corneal endothelial cells.

In the third embodiment described above, the measurement target attribute is the luminance distribution of the lens nucleus, the thickness of the lens, and the equatorial diameter of the lens. However, the measurement target attribute may be some of the luminance distribution of the lens nucleus, the thickness of the lens, and the equatorial diameter of the lens. In addition, the measurement target attribute may include another attribute of the lens such as the thickness of the lens nucleus.

In the third embodiment described above, the determination module 513 determines the estimated age of the sample 20 using the correction values according to the age and gender of the subject. However, the determination module 513 may determine the estimated age of the sample 20 without using these correction values. In addition, in the first, second, and fourth embodiments as well, the determination module 513 may determine the estimated age of the sample 20 using the correction values according to the age and gender of the subject.

In the fourth embodiment described above, the measurement target attribute is the eye axis length, the thickness of the lens, and the anterior chamber depth. The estimation system 1 determines the estimated age for each of the eye axis length, the thickness of the lens, and the anterior chamber depth, and outputs the evaluation information indicating that there is a possibility of presbyopia in the sample 20 in a case where each of the difference values obtained by subtracting the actual age from the determined estimated ages is equal to or more than the predetermined age threshold value.

However, the measurement target attribute does not have to be all of the eye axis length, the thickness of the lens, and the anterior chamber depth as long as the measurement target attribute is at least one of the thickness of the lens or the anterior chamber depth, and the eye axis length. For example, the measurement target attribute may be any one of the thickness of the lens or the anterior chamber depth, and the eye axis length. In this case, the determination module 513 may determine the estimated age for each of any one of the thickness of the lens or the anterior chamber depth, and the eye axis length. Then, in a case where each of the difference values obtained by subtracting the actual age from the estimated ages for any one of the thickness of the lens or the anterior chamber depth, and the eye axis length is equal to or more than the predetermined age threshold value, the output control module 514 may generate and output the evaluation information indicating that there is a possibility of presbyopia in the sample 20.

In the fourth embodiment described above, the measurement target attribute is the eye axis length that is the dimension value of the eye to be examined, the thickness of the lens that is the characteristic of the lens, and the anterior chamber depth that is the characteristic of the anterior chamber. However, the measurement target attribute may be any of the dimension value of the eye to be examined, the characteristic of the lens, and the characteristic of the anterior chamber. For example, the measurement target attribute may be the eye axis length of the eye to be examined. In addition, the dimension value of the eye to be examined as the measurement target attribute may be the vertical width, the horizontal width, and the like of the eye to be examined.

In the fourth embodiment described above, the determination module 513 determines the estimated age of the sample 20 for each of the plurality of measurement target attributes (the eye axis length, the thickness of the lens, and the anterior chamber depth). However, the determination module 513 may determine one estimated age in consideration of the plurality of measurement target attributes. For example, similarly to the above-described fourth embodiment, the determination module 513 may obtain the estimated age for each of the eye axis length, the thickness of the lens, and the anterior chamber depth, weighted-average the obtained three estimated ages with a predetermined weight, and determine the obtained weighted average as the estimated age in consideration of all the eye axis length, the thickness of the lens, and the anterior chamber depth. In addition, the determination module 513 may use information indicating a correspondence between the plurality of measurement target attributes and the biological age as the correspondence information 521 as described below. That is, the determination module 513 may acquire an age corresponding to the measurement values of the plurality of measurement target attributes of the sample 20 from the correspondence information 521, and determine the acquired age as the estimated age. In such a case, in a case where the plurality of measurement target attributes are at least one of the thickness of the lens or the anterior chamber depth, and the eye axis length, the output control module 514 may generate and output, as the evaluation information, information indicating that there is a possibility of presbyopia in the sample 20 when a difference value obtained by subtracting the actual age of the sample 20 from the estimated age determined by the determination module 513 is equal to or more than the predetermined threshold value.

In each of the embodiments described above, the correspondence information 521 is the information in which the information on the value of the attribute of the eye to be examined and the information on the biological age are associated with each other. However, the correspondence information 521 may be another information as long as the information indicates the correspondence between the value of the attribute of the eye to be examined and the biological age. For example, the correspondence information 521 may be information on a model for obtaining the biological age from the value of the attribute of the eye to be examined (for example, a mathematical formula or the like for obtaining the biological age from the value of the attribute of the eye to be examined). For example, in a case where the correspondence information 521 is information on the mathematical formula, the determination module 513 may determine the estimated age of the sample 20 for this attribute by applying the measurement value of the attribute of the sample 20 to this mathematical formula.

In each of the embodiments described above, the output control module 514 outputs the evaluation information indicating the possibility of an abnormality in the measurement target attribute of the sample 20 in a case where the value obtained by subtracting the actual age of the sample 20 from the estimated age of the sample 20 is equal to or more than the predetermined age threshold value. However, the output control module 514 may output information different from the information indicating the possibility of an abnormality in the sample 20 as the evaluation information of the sample 20 on the basis of the estimated age of the sample 20 and the actual age of the sample 20. For example, in a case where the estimated age of the sample 20 is equal to or less than the actual age of the sample 20, the output control module 514 may generate and output evaluation information (for example, text information such as "normal") indicating that the sample 20 is normal.

In each of the embodiments described above, the estimation system 1 determines the estimated age of the sample 20 using any of the characteristic of the cornea, the characteristic of the corner angle, the characteristic of the lens, the characteristic of the anterior chamber, and the dimension value of the eye to be examined as the measurement target attribute. However, the measurement target attribute may be at least one or more of a plurality of characteristics of the anterior segment and a plurality of dimension values of the eye to be examined, and is not limited to the above-described embodiments. For example, the estimation system 1 may determine the estimated age of the sample 20 using two or more of the characteristic of the cornea, the characteristic of the corner angle, the characteristic of the lens, the characteristic of the anterior chamber, and the dimension value of the eye to be examined as the measurement target attribute. For example, the determination module 513 may determine the respective estimated ages similar to those of the first to fourth embodiments, and determine a final estimated age of the sample 20 on the basis of the determined estimated ages. The determination module 513 may determine, as the final estimated age of the sample 20, a weighted average that can be obtained by weighted-averaging the estimated ages similar to those of the first to fourth embodiments with a predetermined weight. The weight in this case is determined as, for example, a value according to the degree of contribution to the eyesight of each measurement target attribute. In addition, the weight in this case may be an equal value. As a result, the estimation system 1 can determine the estimated age of the eye to be examined in consideration of the plurality of characteristics.

In each of the embodiments described above, the measurement value acquisition module 512 acquires the measurement value of the measurement target attribute of the sample 20 from the tomographic image of the sample 20 photographed using the optical coherence tomography. However, the measurement value acquisition module 512 may acquire the measurement value of the measurement target attribute of the sample 20 by another method. For example, the measurement value acquisition module 512 may acquire the measurement value of the measurement target attribute of the sample 20 by receiving specification of the measurement value by the operation section 540.

In each of the embodiments described above, the estimation system 1 is the optical coherence tomography apparatus that obtains the tomographic image of the anterior segment of the eye to be examined by the optical coherence tomography. However, the estimation system 1 may be different from the optical coherence tomography apparatus that obtains the tomographic image of the anterior segment. For example, the estimation system 1 may be another ophthalmic apparatus (for example, an anterior segment photographing apparatus, a corneal shape measurement apparatus, a corneal endothelial cell measurement apparatus, a fundus three-dimensional OCT, and the like), a general-purpose computer, and the like.

### REFERENCE SIGNS LIST

- 1: Estimation system
- 10: Wavelength sweep light source
- 20: Sample
- 100: OCT interference system
- 104: Measurement-side circulator
- 105: Reference-side circulator
- 110: Balanced detector
- 120: Polarization controller
- 200: Alignment optical system
- 201: Collimator lens
- 202: Galvano scanner
- 203: Hot mirror
- 204: Objective lens
- 205a, 205b: White light source
- 206: Beam splitter
- 207: Image forming lens
- 208: Area sensor
- 210: Fixation target light source
- 211: Variable focus movable lens
- 212: Cold mirror
- 213: Hot mirror
- 214: Relay lens
- 215: Beam splitter
- 216: XY position detection light source
- 217: Image forming lens
- 218: Two-dimensional position sensor
- 219: Z position detection light source
- 220: Image forming lens
- 221: Line sensor
- 300: Reference optical system
- 301: Reference section
- 400: k-clock generation interference optical system
- 500: Control unit
- 510: Control section
- 511: Tomographic image acquisition module
- 512: Measurement value acquisition module
- 513: Determination module
- 514: Output control module
- 520: Storage medium
- 521: Correspondence information
- 530: Display section
- 540: Operation section
- 701: Anterior surface
- 702: Lens nucleus
- 703: Anterior surface
- 704: Posterior surface
- 705: Posterior surface
- 706: Lens cortex

## Claims

1. An estimation system comprising:
an acquisition module configured to acquire a measurement value of an attribute of an eye to be examined, the attribute being at least one of a plurality of characteristics of an anterior segment and a plurality of dimension values of the eye to be examined; and
a determination module configured to determine an estimated age of the eye to be examined on a basis of the measurement value and correspondence information indicating a correspondence between a value of the attribute and an age of an eyeball.

2. The estimation system according to claim 1, further comprising an output control module configured to output evaluation information of the eye to be examined on a basis of the estimated age and an actual age that is an actual age of the eye to be examined.

3. The estimation system according to claim 2, wherein the output control module outputs, as the evaluation information, information indicating that there is a possibility of an abnormality in the eye to be examined in a case where the estimated age is greater than the actual age by an age threshold value or more.

4. The estimation system according to claim 3, wherein
the attribute is an astigmatism axis angle, and
the output control module outputs, as the evaluation information, information indicating that there is a possibility of cataract or presbyopia in the eye to be examined in a case where the estimated age is greater than the actual age by the age threshold value or more.

5. The estimation system according to claim 3, wherein
the attribute is a characteristic of a corner angle, and
the output control module outputs, as the evaluation information, information indicating that there is a possibility of an abnormality in the corner angle of the eye to be examined in a case where the estimated age is greater than the actual age by the age threshold value or more.

6. The estimation system according to claim 3, wherein
the attribute is a characteristic of a lens, and
the output control module outputs, as the evaluation information, information indicating that there is a possibility of cataract in the eye to be examined in a case where the estimated age is greater than the actual age by the age threshold value or more.

7. The estimation system according to claim 3, wherein
the attribute is at least one of a thickness of a lens or an anterior chamber depth, and an eye axis length,
the acquisition module acquires the measurement value for each of the at least one and the eye axis length,
the determination module determines the estimated age for the attribute on a basis of the measurement value and the correspondence information with respect to each of the at least one and the eye axis length, and
the output control module outputs, as the evaluation information, information indicating that there is a possibility of presbyopia in the eye to be examined in a case where the estimated age is greater than the actual age by the age threshold value or more with respect to each of the at least one and the eye axis length.

8. The estimation system according to any one of claims 1 to 3, wherein
a plurality of the attributes are an astigmatism axis angle, a characteristic of a corner angle, a characteristic of a lens, an anterior chamber depth, and an eye axis length,
the acquisition module acquires the measurement value for each of the plurality of attributes, and
the determination module determines the estimated age for the attribute on a basis of the measurement value and the correspondence information with respect to each of the plurality of attributes, and determines the final estimated age of the eye to be examined on a basis of the plurality of determined estimated ages.

9. An estimation method executed by an estimation system, the method comprising:
an acquisition step of acquiring a measurement value of an attribute of an eye to be examined, the attribute being at least one of a plurality of characteristics of an anterior segment and a plurality of dimension values of the eye to be examined; and
a determination step of determining an estimated age of the eye to be examined on a basis of the measurement value and correspondence information indicating a correspondence between a value of the attribute and an age of an eyeball.

10. A program for causing a computer to execute:
an acquisition step of acquiring a measurement value of an attribute of an eye to be examined, the attribute being at least one of a plurality of characteristics of an anterior segment and a plurality of dimension values of the eye to be examined; and
a determination step of determining an estimated age of the eye to be examined on a basis of the measurement value and correspondence information indicating a correspondence between a value of the attribute and an age of an eyeball.
